# EUROPEAN PATENT SPECIFICATION

(11) **EP 2 497 447 B1**
(45) Date of publication and mention of the grant of the patent: **19.03.2014**
(21) Application number: 12275004.5
(22) Date of filing: 19.01.2012
(51) Int. Cl.: A61F 2/95

(54) **Introducer assembly and carrier element for a medical device**
Einführereinheit und Trägerelement für eine medizinische Vorrichtung
Ensemble d'introducteur et élément de support pour dispositif médical

(30) Priority: 08.03.2011 GB 201103893
(43) Date of publication of application: 12.09.2012
(73) Proprietor: Cook Medical Technologies LLC, Bloomington, IN 47402-0489 (US)
(72) Inventor: Oehlenschlaeger, Bent, DK-4623 Ll. Skensved (DK); Jensen, Kim, 2000 Frederiksberg (DK)
(74) Representative: Georgiou, Sarah Caroline

(56) References cited:
- WO-A1-2006/105500
- US-A1- 2003 163 156
- US-A1- 2004 015 224
- US-A1- 2005 154 443
- US-A1- 2009 157 162
- US-A1- 2009 287 292
- US-A1- 2011 137 402

## Description

### Technical Field

The present invention relates to an introducer assembly and to a carrier element for deploying an implantable medical device within a patient, in the preferred embodiments for deploying devices for treating aneurysms or Type-A dissections.

### Background of the Invention

The practice of deploying implantable medical devices endoluminally is well established, primarily due to the efficiency of the procedure and minimisation of trauma to the patient. Introducer assemblies used for the deployment of such devices tend to be elongate catheter based assemblies which have provision at their distal ends to hold a medical device in a radially compressed configuration. The medical device may be deployed from the introducer assembly by self-expansion or by means of a separate expansion mechanism, for instance by means of an inflatable balloon.

The design, shape and size of the medical device is best determined for the particular therapeutic application and patient physiology, with the result that for best practice a large number of different devices may be available for use by the surgeon or clinician.

Typically, the medical device is constrained on the introducer at the proximal and distal ends of the device and released by activation of one or more trigger mechanisms.

As a result of the nature of the introducer and the mode of deployment, it can be difficult to ensure precise positioning of the medical device within the patient, particularly in the case of devices which are self-expanding as these tend to spring out of their constrained configurations as soon as the trigger mechanism is actuated. While in many cases this can be catered for by the clinician during the procedure, in particular where it is not necessary to effect extremely precise positioning or orientation of the medical device, there are many instances in which this can cause positioning and deployment difficulties.

These difficulties can be mitigated by providing for sequential release of the medical device, for instance by holding the medical device at one or more intermediate points along its length in addition to at its ends. Sequential release of the device can substantially reduce its sudden and uncontrolled movement from its constrained configuration on the introducer and can also give the clinician a degree of placement control during the expansion of the device.

Some examples of prior art introducers with intermediate constraining mechanisms can be found in US-2002/0151953, US-2002/0040236 and US-2002/0173837. A device according to the preamble of claim 1 is known from the document US-A-2009/287292.

Given that there is a large range of medical devices which differ both in shape, configuration and in dimensions, the design of introducers suitable for a large variety of medical devices is complex.

### Disclosure of the Invention

The present invention seeks to provide an improved introducer assembly for a deploying an implantable medical device and an improved carrier element for such an introducer assembly.

According to an aspect of the present invention, there is provided a medical device carrier for an introducer assembly, the carrier including an elongate element provided with a first diameter and a zone extending along at least a portion thereof for carrying an implantable medical device; and at least one collar on said elongate element in said zone, the or each collar having a second diameter greater than said first diameter and including at least one restraining element configured to restrain releasably a restraining wire.

The or each collar is in the form of a sleeve fitted to the elongate element. The elongate element maybe, for example, a cannula or a catheter.

Advantageously, the or each restraining element is one of a bore and a recess in the sleeve or collar. Preferably, the or each sleeve or collar has at least one end which tapers from said second diameter to substantially said first diameter.

In the preferred embodiment, the or each restraining element is provided in the tapering portion or portions of the or each sleeve or collar.

Advantageously, the sleeve or sleeves are made of a heat shrinkable material.

The sleeve or sleeves may be immovable on the elongate element.

The or each sleeve or collar may have a length from 3 to 10 millimetres, preferably of between 4 and 6 millimetres. The sleeves or collars may be spaced from one another by 5 to 20 millimetres, preferably by 5 to 10 millimetres.

Advantageously, the or each sleeve or collar has a diameter no more than around 50% greater than the diameter of the elongate element. Preferably, the or each sleeve or collar has a height of around 1 millimetre.

According to another aspect of the present invention, there is provided an introducer assembly including an elongate carrier element including a zone extending along at least a portion thereof for carrying an implantable medical device, said carrier element having a first diameter; at least one collar on said carrier element in said zone, the or each collar having a second diameter greater than said first diameter and including at least one restraining element; and at least one restraining wire for restraining a medical device on the carrier element, said at least one restraining wire being releasably restrained by at least one of said restraining elements.

Preferably, the or each restraining wire is a friction fit in at least one of said restraining elements. The or each restraining wire may be restrained by a plurality of said collars, or sleeves.

In a practical embodiment, an implantable medical device is securable to said carrier element in said zone and held to the carrier element by at least one restraining wire, the or each restraining wire being restrained by said restraining elements so as to restrain said medical device at a plurality of positions along its length.

Preferably, the device is held to said carrier element by being restrained internally by said restraining wire or wires.

According to another aspect of the invention there is provided a method of forming an introducer assembly, the method including the steps of: providing an elongate carrier element, the carrier element including a zone extending along at least a portion thereof for carrying an implantable medical device, said carrier element having a first diameter; and providing on said carrier element at selected positions in said zone at least one collar, wherein the or each collar is a sleeve formed from a heat shrinkable material, the or each sleeve having a second diameter greater than said first diameter and including at least one restraining element configured to restrain releasably a restraining wire; and bonding the or each sleeve to the elongate element by heat shrinking.

Advantageously, the method includes the step of fitting an implantable medical device to said carrier element in said zone and holding said device to the carrier element by at least one restraining wire, the or each retraining wire being restrained by said restraining elements and said medical device being retrained at a plurality of positions along its length. The device is preferably held to said carrier element by being restrained internally by said restraining wire or wires.

### Brief Description of the Drawings

Embodiments of the present invention are described below, by way of example only, with reference to the accompanying drawings, in which:
Figure 1 is a side elevational view of a carrier element of an embodiment of introducer assembly;
Figure 2 is a side elevational view in enlarged view of a sleeve of the carrier element of Figure 1;
Figure 3 is an end view of the sleeve of Figure 2;
Figure 4 is a view of the carrier element of Figure 1 with an implantable medical device loosely coupled thereto;
Figure 5 is a schematic diagram of an example of introducer assembly comprising a carrier element of the type shown in Figures 1 and.

### Description of the Preferred Embodiments

The elements of the drawings are not shown to scale and often enlarged in order to be able to show better the elements of the described embodiments.

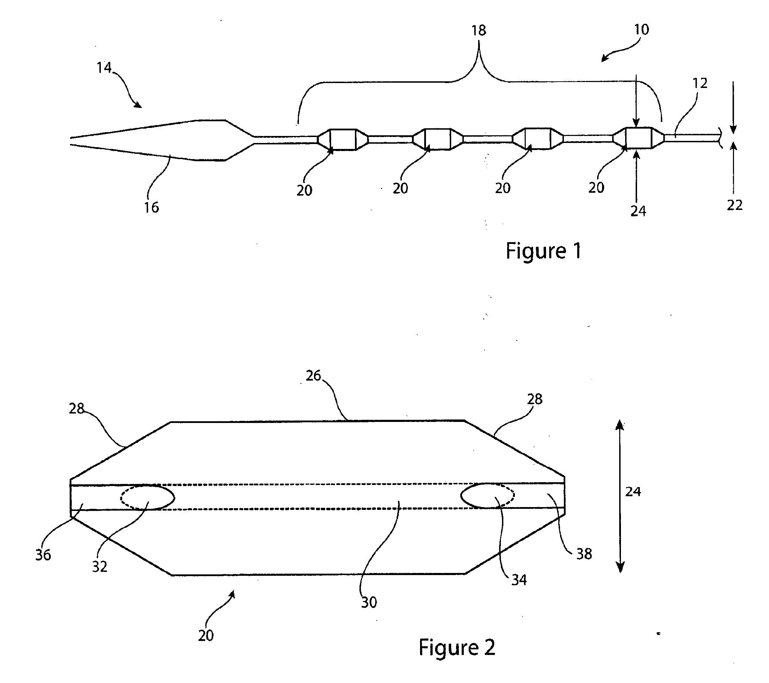

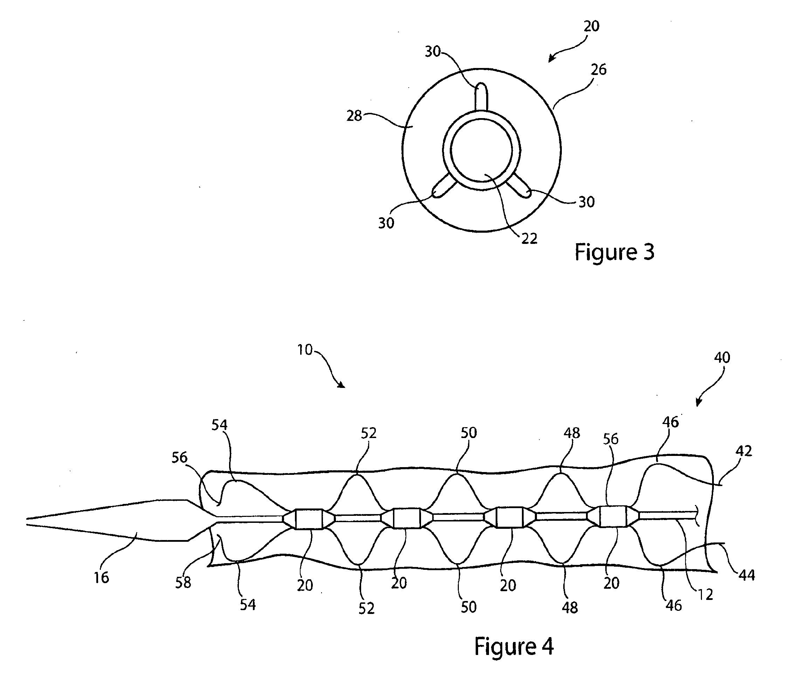

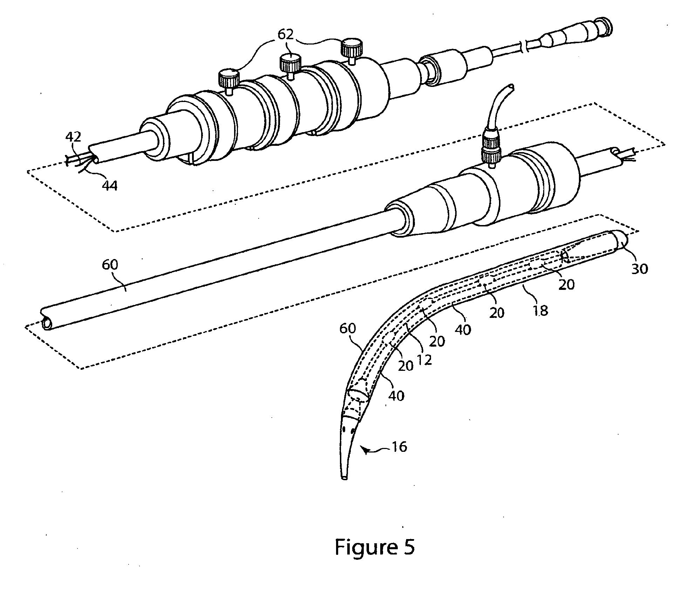

Referring to Figure 1 there is shown the distal end of an embodiment of carrier element 10 used for carrying an implantable medical device in an introducer assembly. The carrier includes an elongate element 12, which is typically a catheter or cannula. The elongate element 12 typically extends from a proximal manipulation end of the introducer assembly (not visible in Figure 1 but visible in Figure 5) to a distal end 14, typically provided a dilator tip 16 of conventional form. Shown particularly in Figure 1 is the zone 18 of the carrier element 10 at which an implantable medical device (shown in Figures 4 and 5) is held and compressed radially for insertion into the lumen of a patient.

The carrier 10, at the zone 18, is provided, in this embodiment, with a plurality of sleeves 20 which are fitted on the outside of the elongate element 12. There may be provided any number of sleeves 20, this primarily being dependent upon the nature and structure of the medical device to be carried on the carrier 10 and the extent to which it is desired to restrain the medical device at a plurality of points along its length.

The elongate element 12, which typically is circular or annular in axial cross-section, has a first diameter 22, whereas the or each sleeve 20, which is also generally annular in axial cross-section, has a maximum or second diameter 24 which is greater than the diameter 22 of the element 12.

The elongate element 12, in the preferred embodiment, includes a lumen extending longitudinally therein, which is substantially aligned with or a continuation of a lumen extending longitudinally within the dilator tip 16. This lumen allows for the passage of a guide wire through the carrier 10 in conventional manner.

As can be seen in particular with regard to Figures 2 and 3, the or each sleeve 20, in this embodiment, includes a central body portion 26 which is substantially cylindrical along its length and is bound on either side by tapering ends 28 which reduce in height from the diameter 24 towards the diameter 22 of the elongate element 12. It is not necessary for the body portion 26 to be cylindrical but this is preferred.

Extending within the material of the or each sleeve 20 is at least one bore 30 which extends from one opening 32 in one of the tapering ends 28 to a second opening 34 in the opposite tapering end. Each opening 32, 34 is preferably in the form of a scalloped entry point 36, 38, respectively. In some embodiments there may be provided a single lumen or bore 30 within the or each sleeve 20 but in the preferred embodiments, as shown in Figure 3, there is preferably provided a plurality of, most preferably three, bores 30 spaced substantially evenly around the central bore 22 of the or each sleeve 20. It will be appreciated that the central bore 22 is sized to receive the elongate element 12.

Thus, with reference to Figure 1, where a plurality of sleeves 20 are located on the elongate element 12, these will provide a plurality of sets of bores or channels 30 arranged along the length of the zone 18. The bores 30 of adjacent sleeves 20 are preferably aligned with one another, although this is not necessary.

Not shown in the Figures, for the purposes of simplification of the drawings only, are restraining devices for use in restraining the proximal and the distal ends of a medical device. These restraining devices are well known in the art and are not described in any detail herein. In some cases, for example, there may be provided within the dilator tip 16 one or more bores for receiving one or more trigger wires used for restraining the proximal end of the medical device. It is not excluded, however, that the sleeves 20 can in some embodiments, provide all of the mechanisms for restraining a medical device, in which case the medical device would have its proximal and distal ends located adjacent a respective sleeve 20, with one or more additional sleeves being located at positions intermediate the two end sleeves.

The or each sleeve is made of a heat shrinkable material. The use of a different material for the sleeve or sleeves 20 enables carrier element 12 and sleeves 20 to be optimised for their particular functions. For instance, the carrier element 12 can be made so as to provide flexibility of the introducer assembly and support of a medical device, while the sleeves 20 can be formed of a material which is optimal for the necessary restraining characteristics provided by the sleeves 20. Similarly, making the sleeves 20 of a different material provides convenient manufacturing characteristics, such as enabling the fitting of the sleeves onto the carrier element 12 after this has been fabricated, by, heat shrinking.

The or each sleeve 20 is preferably of a relatively small dimension, in some embodiments having a length ranging from 3 to 10mm and in preferred embodiments having a length of between 4 to 6mm. The sleeves can thus be considered small compared to the length of the carrier element and/or the implantable medical device. This minimises any effect of the sleeves 20 on the characteristics of the introducer assembly, such as flexibility of the carrier element 12.

It is preferred that the diameter of the or each sleeve 20 is no more than around 50% greater than the diameter 22 of the elongate carrier element 12. In the preferred embodiment, the or each sleeve 20 extends beyond the radial perimeter of the carrier element 12 by a height of around 1 mm or less.

Where a plurality of sleeves 20 is provided on the carrier element 12, these are preferably spaced from one another, in the preferred embodiments, by between 5 and 20mm and more preferably by between 5 to 10mm. In practice, the spacing between the sleeves 20 may be dependent upon the characteristics of the medical device to be carried on the introducer, such as the number, length, and spacings of any stents or stent rings of the medical device. In the preferred embodiment, there are provided sufficient sleeves 20 to be able to restrain every stent ring of the medical device, although it is envisaged that in other embodiments some of the stent rings of the medical device may be left unconstrained so as to be able to expand radially outwardly even when the medical device is constrained to the carrier element 12 by means of the sleeves 20 and restraining elements coupled thereto. This would enable the medical device to expand to provide a plurality of bulbous portions along its length in what could be described as a semi-deployed configuration, useful in providing gradual expansion of the medical device.

Referring now to Figure 4, there is shown the distal end of the carrier element 10 of Figure 1 with an implantable medical device 40 shown loosely attached thereto. In practice, the medical device 40 would be constrained tight on the carrier element 10, so as to be very close to or in abutment against at least the sleeves 20 and in some instances also against the elongate element 12. The medical device 40 is shown in this semi-expanded form in Figure 4 for the purposes of clarity of visualisation only.

The medical device 40, in this example, may be a stent or stent graft having a generally tubular shape and which extends across the zone 18, from the distal end of the dilator tip 16 to a position along the carrier element 12 which includes a proximal restraining or attachment point for holding the other end of the device 40 against the carrier element 10.

The carrier element 10 is, in this embodiment, designed to be used with a plurality of trigger wires, of which two, 42 and 44, are shown in Figure 4. It will be appreciated that there would typically be as many trigger wires 42-44 as there are series of bores and channels 30 in the sleeves 20. Thus, for the embodiment of Figure 3 having in each sleeve 20 three bores or channels 30, there would typically be three trigger wires in the assembly.

As will be apparent from Figure 4, the arrangement of the trigger wires 42-44 is such that these pass from an external manipulation end of the introducer assembly and typically from a control element thereof, up to the zone 18 of the carrier element 10. At this position 18, the trigger wires 42-44 alternate between being coupled at points 46-54 to the medical device 40 and between these positions into the respective bores or channels 30 of the adjacent sleeves 20. The arrangement could be described as interdigitating between the medical device 10 and the sleeves 20 in an alternating fashion.

The ends 56, 58 of the trigger wires 42, 44, in this embodiment, locate into associated recesses (not shown) in the dilator tip 16, for example in a friction fit. In this manner, these ends 56, 58 are held in place until they are forcibly removed during actuation of the trigger wires 42-44.

The trigger wires 42-44 can be coupled at points 46-54 to the medical device 40 in any suitable way, including through graft material, around stent struts or through appropriate loops, for example of thread, located on the inside of the medical device 40.

In practice, the trigger wires 42-44 will be taut, thereby to pull the medical device 40 radially inwardly towards the carrier element 12 so this rests close to carrier element 12. It will be appreciated, therefore, that the medical device 40 will be constrained not only at its proximal and distal ends, in conventional manner, but also at one or more intermediate positions, in this example four positions, along its length.

The provision of the sleeves 20, which in practice bulge from the carrier element 12, allows use of a simple carrier element, a cannula or catheter for instance, which can have minimal outer diameter. Moreover, the sleeve or sleeves 20, being preferably formed as separate elements fitted to the carrier 12, allows the sleeves 20 to be positioned optimally for the specific medical device 40 to be carried by the introducer assembly. It is not necessary to change any structural elements of the carrier 12 to accommodate different medical devices 40, it being necessary only to chose the number and positions of the sleeves 20. For example, there could be provided as many sleeves 20 as there are stent rings in the medical device 40, while in other embodiments, there could be fewer sleeves 20 than there are stent rings in the medical device, thereby to hold down only some of the stent rings. It is also envisaged that there could be more sleeves 20 than the number of stent rings to restrain, in which case a plurality of sleeves could be used to restrain a single stent ring (for example to restrain both proximal and distal ends of a stent ring).

The arrangements of the preferred embodiments allow for the restraining of the medical device 40 at a plurality of points along its length from within the medical device. In other words the medical device 40 is compressed radially inwardly by pulling from the inside (by the trigger wires 42-44 co-operating with the sleeves 20). This gives a simple and reliable constraining function, particularly compared to structures which seek to constrain the medical device from the outside.

The preferred embodiments, having the restraining elements in the form of a bore or channel in the sleeve, provides a simple restraining mechanism which can be implemented with minimal volume and thus is particularly suited for introducer assemblies and carrier elements 10 of small external diameter.

With reference to Figures 2 to 4 in particular, having entry and exit points 32, 34 for the channels or lumens 30 of the each sleeve 20 located in the conical portions 28 of the sleeves 20, provides convenient location for these points 32, 34 and also avoids any sharp shoulder or edge to the sleeves 20 which could snag on the lumen walls or on the medical device 40 once deployed.

The restraining wires 42-44 are preferably also a friction fit within the channels or lumens 30 so as not to slide out of these until they are positively withdrawn by actuation of the appropriate mechanism by the clinician (described in further detail below).

With reference now to Figure 5, this shows an example of introducer assembly having a structure which is generally known in the art. The assembly includes an outer sheath 60 which normally covers the carrier element 10 and extends up to the proximal end of the dilator tip 16. The assembly is also provided with a proximal actuation section which includes a series of release mechanisms 62 movable upon release of appropriate clamping screws. The release mechanisms are attached to the trigger wires 42-44 and any other wires or trigger elements of the assembly. The release mechanisms 62 can be retracted, in a known manner, to activate the trigger elements.

In connection with the trigger wires 42-44, as the appropriate release mechanism 62 is unlocked and moved backwards (in a proximal direction) by the clinician, this action will pull backwardly the distal ends 56, 58 of the release wires 42-44. First, the wires 42-44 will come out of the respective bores or slots in the dilator tip 16, thereby enabling the end of the medical device 40 at the location 54, to expand outwardly. The remainder of the medical device 40, particularly from locations 52 back to 46, will remain constrained by the trigger wires 42-44, held in friction fit manner within the sleeves 20. Further movement of the associated release mechanism 62 will eventually cause the trigger wires 42-44 to be released from the distal-most sleeve 20 and thus to allow expansion of the next position 52 of the medical device. Continued further withdrawal of the trigger wires 42-44 will thus provide sequential release and expansion of the medical device 40, this being in a much more controlled manner than with prior art mechanisms, particularly those with restrain only the proximal and distal ends of the medical device.

In addition to providing a convenient and efficient restraining arrangement, the sleeves 20 enable easy manufacture and assembly of the introducer and of the carrier element 10, in a configuration optimised for the particular medical device to be carried by the assembly. In a first instance, the characteristics of the medical device and its deployment can be determined, in particular the extent to which this is to be restrained on the introducer assembly and how it is desired to release this from the introducer. This will indicate the appropriate number and spacings of the sleeves 20. The sleeves can then be slid onto the elongate element 12 during its assembly and then bonded thereto, for instance by a bonding agent, by heat shrinking, by welding or the like, at the appropriate locations. It is not necessary to manufacture a different elongate element 12 or different sleeves 20, the only differences from one assembly to another need only be the number and positions of the sleeves 20.

Once the sleeves 20 have been positioned and set on the elongate element 12, the medical device can then be fitted to the device 12 in any conventional manner, typically by feeding/threading the trigger wires 42-44 in the arrangement shown in Figure 4 and then tightening these so as to assist in the radial compression of the medical device 40 to the elongate element 12.

The preferred embodiments use a number of trigger wires 42-44 equivalent to the number of channels or bores 30 in each of the sleeves 20. This provides for sequential deployment of the medical device 40 from its proximal end first (that is the end closest to the dilator tip 16). However, other embodiments are envisaged which provide for different deployment sequences. For example, by use of a plurality of sets of trigger wires 42-44, it is possible to release different ones of the positions 46-54 of the medical device 40 at different times. For instance, by having trigger wires specifically constraining the central section 50 of the medical device 40, this could be released before release of the other parts of the medical device. In this regard, some or all of the sleeves 20 could be provided with more bores or channels 30 to accommodate the trigger wires of the different sets.

In this manner, any number of sets of trigger wires 42-44 could be provided in dependence upon the sequence of deployment desired for the particular medical device 40. It will be appreciated that a greater number of triggers wires 42-44 will increase the volume of material to be held within the introducer assembly.

It is not necessary for the sleeves 20 to be evenly spaced from one another as their spacings could be determined by the particular medical device 40. They could, for example, have uneven spacings, with some of the sleeves 20 being closer to one another relative to others.

Although the preferred embodiments above make use of a channel or lumen 30 within the sleeves 20, in alternative embodiments there could be provided grooves within the sleeves 20 into which trigger wires 42-44 can fit in a friction-fit manner.

It will be appreciated that the dimensions of the sleeves 20 described above are exemplary and that these may change in dependence upon the size of the elongate element 12 and the characteristics of the medical device 40 to be constrained thereby.

## Claims

1. A medical device carrier (10) for an introducer assembly, the carrier including:
an elongate element (12) provided with a first diameter (22) and including a zone (18) extending along at least a portion thereof for carrying an implantable medical device; and
at least one collar on said elongate element in said zone, the or each collar having a second diameter (24) greater than said first diameter (22) **characterised in that** the collar includes at least one restraining element (30) configured to restrain releasably a restraining wire; wherein the or each collar is a sleeve (20) fitted to the elongate element (12) and
wherein the sleeve or sleeves are formed from a heat shrinkable material.

2. A medical device carrier according to claim 1, wherein the elongate element (12) is a cannula or catheter.

3. A medical device carrier according to any preceding claim, wherein the or each restraining element (30) is one of a bore and a recess in the or each sleeve (20).

4. A medical device carrier according to any preceding claim, wherein the or each sleeve (20) has at least one end which tapers from said second diameter (24) to substantially said first diameter (22) and wherein the or each restraining element (30) is preferably provided in the tapering portion or portions of the or each sleeve.

5. A medical device carrier according to any preceding claim, wherein the sleeve or sleeves (20) are made of a material different from a material of the carrier element.

6. A medical device carrier according to any preceding claim, wherein the sleeve or sleeves (20) are formed from a polycarbonate material.

7. A medical device carrier according to any preceding claim, wherein the sleeve or sleeves (20) are immovable on the elongate element (12).

8. A medical device carrier according to any preceding claim, wherein the or each sleeve (20) has a length from 3 to 10 millimetres or preferably of between 4 and 6 millimetres.

9. A medical device carrier according to any preceding claim, including a plurality of sleeves (20), wherein the sleeves are spaced from one another by 5 to 20 millimetres, or preferably by 5 to 10 millimetres.

10. A medical device carrier according to any preceding claim, wherein the sleeve or sleeves (20) have a diameter no more than around 50% greater than the diameter of the elongate element, and preferably have a height of around 1 millimetre.

11. An introducer assembly including:
the medical device carrier of any preceding claim; and
at least one restraining wire (42,44) for restraining a medical device on the carrier element, said at least one restraining wire being releasably restrained by at least one of said restraining elements (30).

12. An introducer assembly according to claim 11, wherein the or each restraining wire (42, 44) is a friction fit in at least one of said restraining elements, (30) and wherein preferably the or each restraining wire (42, 44) is restrained by a plurality of said sleeves (20).

13. An introducer assembly according to any of claims 11-12, wherein an implantable medical device (40) is securable to said carrier element in said zone (18) and held to the carrier element by at least one restraining wire (42, 44), the or each restraining wire being restrained by said restraining elements (30) so as to restrain said medical device (40) at a plurality of positions along its length and wherein the device is preferably held to said carrier element by being restrained internally by said restraining wire or wires (42, 44).

14. A method of forming an introducer assembly according to claim 1, the method including the steps of:
providing an elongate carrier element (12), the carrier element including a zone (18) extending along at least a portion thereof for carrying an implantable medical device, said carrier element having a first diameter (22); and
providing on said carrier element at selected positions in said zone (18) at least one collar, wherein the or each collar is a sleeve (20) formed from a heat shrinkable material, the or each sleeve having a second diameter (24) greater than said first diameter and including at least one restraining element (30) configured to restrain releasably a restraining wire; and
bonding the or each sleeve (20) to the elongate element (12) by heat shrinking.

15. A method according to claim 14, including the step of fitting an implantable medical device (40) to said carrier element in said zone (18) and holding said device to the carrier element by at least one restraining wire (42, 44), the or each retraining wire being restrained by said restraining elements (30) and said medical device (40) being retrained at a plurality of intermediate positions along its length wherein the device is preferably held to said carrier element by being restrained internally by said restraining wire or wires.

## Patentansprüche

1. Trägerelement (10) für eine medizinische Vorrichtung für eine Einführereinheit, wobei das Trägerelement Folgendes aufweist:
ein längliches Element (12) mit einem ersten Durchmesser (22) und einem sich entlang mindestens einem Abschnitt davon erstreckenden Bereich (18) zum Tragen einer implantierbaren medizinischen Vorrichtung und
mindestens einen Bund an dem länglichen Element in dem Bereich, wobei der oder jeder Bund einen zweiten Durchmesser (24) hat, der größer als der erste Durchmesser (22) ist, **dadurch gekennzeichnet, dass** der Bund mindestens ein Rückhalteelement (30) aufweist, das dazu konfiguriert ist, einen Rückhaltedraht freigebbar zurückzuhalten, wobei der oder jeder Bund eine Hülse (20) ist, die an dem länglichen Element (12) angebracht ist, und
wobei die Hülse oder Hülsen aus einem aufschrumpfbaren Material gebildet sind.

2. Trägerelement für eine medizinische Vorrichtung nach Anspruch 1, wobei das längliche Element (12) eine Kanüle oder ein Katheter ist.

3. Trägerelement für eine medizinische Vorrichtung nach einem der vorhergehenden Ansprüche, wobei das oder jedes Rückhalteelement (30) eine Bohrung oder eine Aussparung in der oder jeder Hülse (20) ist.

4. Trägerelement für eine medizinische Vorrichtung nach einem der vorhergehenden Ansprüche, wobei die oder jede Hülse (20) mindestens ein Ende hat, das sich von dem zweiten Durchmesser (24) zu im Wesentlichen dem ersten Durchmesser (22) hin verjüngt und wobei das oder jedes Rückhalteelement (30) vorzugsweise in dem sich verjüngenden Abschnitt oder den sich verjüngenden Abschnitten der oder jeder Hülse vorgesehen ist.

5. Trägerelement für eine medizinische Vorrichtung nach einem der vorhergehenden Ansprüche, wobei die Hülse oder Hülsen (20) aus einem Material hergestellt sind, das sich von einem Material des Trägerelements unterscheidet.

6. Trägerelement für eine medizinische Vorrichtung nach einem der vorhergehenden Ansprüche, wobei die Hülse oder Hülsen (20) aus einem Polycarbonatmaterial gebildet sind.

7. Trägerelement für eine medizinische Vorrichtung nach einem der vorhergehenden Ansprüche, wobei die Hülse oder Hülsen (20) an dem länglichen Element (12) unbeweglich sind.

8. Trägerelement für eine medizinische Vorrichtung nach einem der vorhergehenden Ansprüche, wobei die oder jede Hülse (20) eine Länge von 3 bis 10 Millimetern oder vorzugsweise 4 bis 6 Millimetern hat.

9. Trägerelement für eine medizinische Vorrichtung nach einem der vorhergehenden Ansprüche, das mehrere Hülsen (20) aufweist, wobei die Hülsen 5 bis 20 Millimeter oder vorzugsweise 5 bis 10 Millimeter voneinander beabstandet sind.

10. Trägerelement für eine medizinische Vorrichtung nach einem der vorhergehenden Ansprüche, wobei die Hülse oder Hülsen (20) einen Durchmesser haben, der höchstens ungefähr 50% größer als der Durchmesser des länglichen Elements ist, und vorzugsweise eine Höhe von ungefähr 1 Millimeter haben.

11. Einführereinheit, die Folgendes aufweist:
das Trägerelement für eine medizinische Vorrichtung nach einem der vorhergehenden Ansprüche und
mindestens einen Rückhaltedraht (42, 44) zum Zurückhalten einer medizinischen Vorrichtung am Trägerelement, wobei der mindestens eine Rückhaltedraht durch mindestens eines der Rückhalteelemente (30) freigebbar zurückgehalten wird.

12. Einführereinheit nach Anspruch 11, wobei der oder jeder Rückhaltedraht (42, 44) reibschlüssig in mindestens einem der Rückhalteelemente (30) angeordnet ist und wobei vorzugsweise der oder jeder Rückhaltedraht (42, 44) durch mehrere der Hülsen (20) zurückgehalten wird.

13. Einführereinheit nach einem der Ansprüche 11 - 12, wobei eine implantierbare medizinische Vorrichtung (40) an dem Trägerelement in dem Bereich (18) befestigt werden kann und durch mindestens einen Rückhaltedraht (42, 44) an dem Trägerelement gehalten wird, wobei der oder jeder Rückhaltedraht so durch die Rückhalteelemente (30) zurückgehalten wird, dass die medizinische Vorrichtung (40) an mehreren Positionen entlang ihrer Länge zurückgehalten wird und wobei die Vorrichtung vorzugsweise an dem Trägerelement gehalten wird, indem sie innen durch den Rückhaltedraht oder -drähte (42, 44) zurückgehalten wird.

14. Verfahren zum Bilden einer Einführereinheit nach Anspruch 1, wobei das Verfahren die folgenden Schritte aufweist:
Bereitstellen eines länglichen Trägerelements (12) mit einem sich entlang mindestens einem Abschnitt davon erstreckenden Bereich (18) zum Tragen einer implantierbaren medizinischen Vorrichtung, wobei das Trägerelement einen ersten Durchmesser (22) hat, und
Bereitstellen mindestens eines Bundes an dem Trägerelement an ausgewählten Positionen im Bereich (18), wobei der oder jeder Bund eine aus einem aufschrumpfbaren Material gebildete Hülse (20) ist, wobei die oder jede Hülse einen zweiten Durchmesser (24) hat, der größer als der erste Durchmesser ist, und mindestens ein Rückhalteelement (30) aufweist, das dazu konfiguriert ist, einen Rückhaltedraht freigebbar zurückzuhalten, und
Bonden der oder jeder Hülse (20) an das längliche Element (12) durch Aufschrumpfen.

15. Verfahren nach Anspruch 14, das den Schritt des Anbringens einer implantierbaren medizinischen Vorrichtung (40) an das Trägerelement in dem Bereich (18) und des Haltens der Vorrichtung an dem Trägerelement durch mindestens einen Rückhaltedraht (42, 44) aufweist, wobei der oder jeder Rückhaltedraht durch die Rückhalteelemente (30) zurückgehalten wird und die medizinische Vorrichtung (40) an mehreren Zwischenpositionen entlang ihrer Länge zurückgehalten wird, wobei die Vorrichtung vorzugsweise an dem Trägerelement gehalten wird, indem sie innen durch den Rückhaltedraht oder -drähte zurückgehalten wird.

## Revendications

1. Support pour dispositif médical (10) pour un ensemble d'introducteur, le support comprenant :
un élément allongé (12) pourvu d'un premier diamètre (22) et comportant une zone (18) s'étendant le long d'au moins une portion de celui-ci pour supporter un dispositif médical implantable ; et
au moins un collier sur ledit élément allongé dans ladite zone, le ou chaque collier ayant un deuxième diamètre (24) supérieur audit premier diamètre (22), **caractérisé en ce que** le collier comporte au moins un élément de retenue (30) configuré pour retenir, de manière amovible, un fil de retenue ; le ou chaque collier étant un manchon (20) assujetti à l'élément allongé (12) et
le manchon ou les manchons étant formés d'un matériau thermorétractable.

2. Support pour dispositif médical selon la revendication 1, dans lequel l'élément allongé (12) est une canule ou un cathéter.

3. Support pour dispositif médical selon l'une quelconque des revendications précédentes, dans lequel le ou chaque élément de retenue (30) est l'un parmi un alésage et un retrait dans le ou chaque manchon (20).

4. Support pour dispositif médical selon l'une quelconque des revendications précédentes, dans lequel le ou chaque manchon (20) présente au moins une extrémité qui diminue sous forme conique depuis ledit deuxième diamètre (24) jusqu'à substantiellement ledit premier diamètre (22) et dans lequel le ou chaque élément de retenue (30) est de préférence prévu dans la portion ou les portions de forme conique du ou de chaque manchon.

5. Support pour dispositif médical selon l'une quelconque des revendications précédentes, dans lequel le ou les manchons (20) sont fabriqués en un matériau différent d'un matériau de l'élément de support.

6. Support pour dispositif médical selon l'une quelconque des revendications précédentes, dans lequel le ou les manchons (20) sont formés d'un matériau à base de polycarbonate.

7. Support pour dispositif médical selon l'une quelconque des revendications précédentes, dans lequel le ou les manchons (20) sont disposés de manière immuable sur l'élément allongé (12).

8. Support pour dispositif médical selon l'une quelconque des revendications précédentes, dans lequel le ou les manchons (20) ont une longueur de 3 à 10 millimètres ou de préférence comprise entre 4 et 6 millimètres.

9. Support pour dispositif médical selon l'une quelconque des revendications précédentes, comportant une pluralité de manchons (20), les manchons étant espacés les uns des autres de 5 à 20 mm, de préférence de 5 à 10 mm.

10. Support pour dispositif médical selon l'une quelconque des revendications précédentes, dans lequel le ou les manchons (20) ont un diamètre ne dépassant pas environ 50 % de plus que le diamètre de l'élément allongé, et ont de préférence une hauteur d'environ 1 mm.

11. Ensemble d'introducteur comportant :
le support pour dispositif médical selon l'une quelconque des revendications précédentes ; et
au moins un fil de retenue (42, 44) pour retenir un dispositif médical sur l'élément de support, ledit au moins un fil de retenue étant retenu de manière amovible par au moins l'un desdits éléments de retenue (30).

12. Ensemble d'introducteur selon la revendication 11, dans lequel le ou chaque fil de retenue (42, 44) est ajusté par friction dans au moins l'un desdits éléments de retenue (30) et dans lequel de préférence le ou chaque fil de retenue (42, 44) est retenu par une pluralité desdits manchons (20).

13. Ensemble d'introducteur selon l'une quelconque des revendications 11 à 12, dans lequel un dispositif médical implantable (40) peut être fixé audit élément de support dans ladite zone (18) et être retenu sur l'élément de support par au moins un fil de retenue (42, 44), le ou chaque fil de retenue étant retenu par lesdits éléments de retenue (30) de manière à retenir ledit dispositif médical (40) dans une pluralité de positions le long de sa longueur et dans lequel le dispositif est de préférence retenu sur ledit élément de support en étant retenu intérieurement par ledit ou lesdits fils de retenue (42, 44).

14. Procédé de formation d'un ensemble d'introducteur selon la revendication 1, le procédé comportant les étapes suivantes :
fournir un élément de support allongé (12), l'élément de support comportant une zone (18) s'étendant le long d'au moins une portion de celui-ci pour supporter un dispositif médical implantable, ledit élément de support ayant un premier diamètre (22) ; et
fournir, sur ledit élément de support, en des positions sélectionnées dans ladite zone (18) au moins un collier, le ou chaque collier étant un manchon (20) formé d'un matériau thermorétractable, le ou chaque manchon ayant un deuxième diamètre (24) supérieur audit premier diamètre et comportant au moins un élément de retenue (30) configuré pour retenir de manière amovible un fil de retenue ; et
coller le ou chaque manchon (20) à l'élément allongé (12) par retrait thermique.

15. Procédé selon la revendication 14, comportant l'étape consistant à assujettir un dispositif médical implantable (40) audit élément de support dans ladite zone (18) et à retenir ledit dispositif à l'élément de support par au moins un fil de retenue (42, 44), le ou chaque fil de retenue étant retenu par lesdits éléments de retenue (30) et ledit dispositif médical (40) étant retenu dans une pluralité de positions intermédiaires le long de sa longueur et dans lequel le dispositif est de préférence retenu sur ledit élément de support en étant retenu intérieurement par ledit ou lesdits fils de retenue.
